# EUROPEAN PATENT SPECIFICATION

(11) **EP 2 130 559 B1**
(45) Date of publication and mention of the grant of the patent: **20.06.2018**
(21) Application number: 09011199.8
(22) Date of filing: 16.12.2005
(51) Int. Cl.: A61M 5/145

(54) **Syringe pump and docking station mountable on an IV pole**
Spritzenpumpe und Andockstation zur Montage an einen Infusionsständer
Pompe à seringue et station d'accueil montable sur un support IV

(43) Date of publication of application: 09.12.2009
(62) Divisional of application: 05027562.7
(73) Proprietor: Barak, Swi, Caesarea 38900 (IL)
(72) Inventor: Barak, Swi, Caesarea 38900 (IL)
(74) Representative: Harrison IP Limited

(56) References cited:
- EP-A- 0 402 553
- EP-A- 1 279 410
- EP-A- 1 329 232
- WO-A-01/30421
- US-A- 5 034 004
- US-A- 5 232 449

## Description

### Field and Background of the Invention

This invention relates to a syringe pump useful for administration of liquids to a patient by a medical pump for example from any size and type of syringe to a patient through a flexible tube.
Systems for administration of liquids to a patient is widely known. However, a variety of different pumps are available for propelling a drug to a patient, which may differ, among others, in the manner and principle in which they operate. E.g. prior art EP 0 402 553 A1 discloses a syringe pump according to the preamble of claim 1, wherein coaxial moving rods which slide over a a fixed rod are used to transfer an electrical signal of a strain gauge signalling the pressure inside the syringe. The present invention is concerned with two aspects for administrating a liquid to a patient. Its first aspect, the invention is concerned with a miniature mechanism for a syringe driver, which enable the system to be portable. The second aspect, the invention is concerned with control sensors for the use with a high precision liquid administration pump.

### Summary of the invention

The invention provides, by a first of its aspects, a miniature syringe pump for propelling drugs through a lumen of a flexible tube segment, the system comprising the miniature pump and a multi-purpose docking station. Once the pump was connected to the docking station, it's converted to be a stationary pump.
The Docking station has a male connector corresponding to a female connector on the pump, through the connector the syringe pump will:
a. Transmit the operation data to the docking station, that will display on a large and readable display.
b. Will charge the pump rechargeable batteries and will supply DC to the pump, while connected to mains.
c. Transmission of data to a central computer memory to create a file that can be tracked down.
d. Will enable connectivity to a central computer for data setting and control parameters.
The syringe pump mechanics comprises a screw bar and 2 guiding bars. The guiding bars will reduce the friction of the actuator to minimum and will assure smooth operation of the mechanics, but in parallel will be used for data transmission wires to the pump controller (micro controller) of the data from a micro switch mounted on the actuator, if the syringe is located on the actuator slot as required.

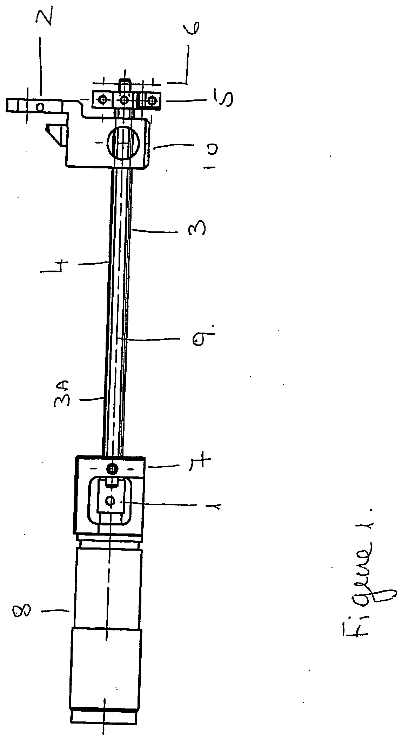

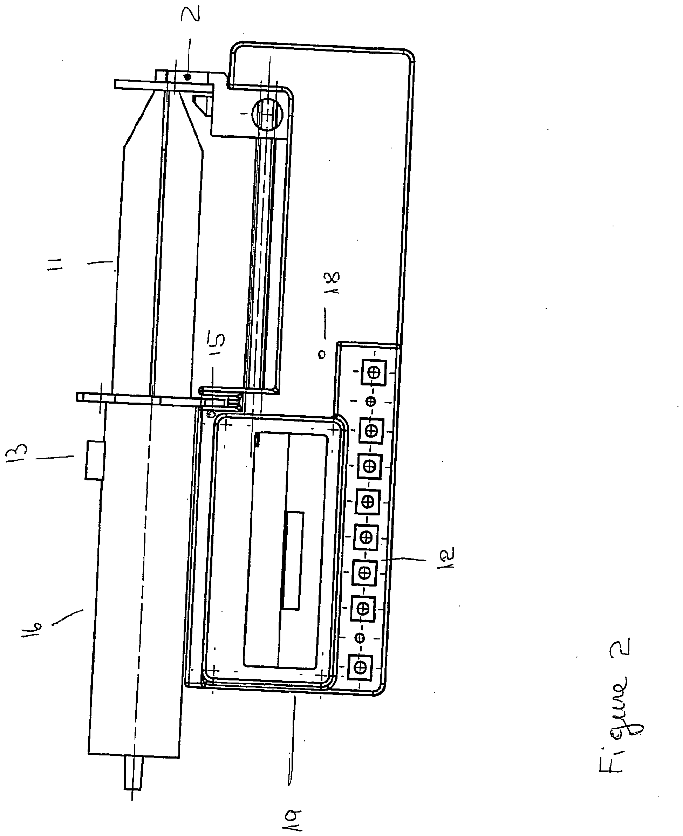

According to one preferred embodiment of the invention, the syringe pump has three sensors to detect the presence of the syringe and it's correct and safe located.
a. Sensor located on the actuator 2, where it's signals are transmitted through the guiding bars 3 and 3A on fig 1
b. Sensor located on the syringe location slot 15 on fig 2
c. Third sensor located on the syringe holder 13 connected to a linear potentiometer activated by a spring, not shown, to sense the diameter of the syringe.

By an improved design of the pump there is further provided with a door, not shown, with locking possibility, to protect any unauthorized access to the syringe.

By another preferred embodiment, the present invention includes a motor 8 fig 1 and an encoder 6, for rotating the screw axis and control the location of the actuator 10.
According to a second aspect, of the present invention, there is provided a motor connected to a bearing (front bearing) 7 which is connected to a screw axis 9 and two guiding bars 3 and 3A that are connected to a second bearing (Rear Bearing) 5. The front bearing 7 has a magnet 1 on the motor axis that counts the number of motor revolutions which are compared with the results of two independent channels counted on the encoder mounted 6.

By another preferred embodiment of the present invention there is provided a motor 8 and a micro-controller, not shown, to control the motor revolution in order to get an improved linear delivery of the liquid and preventing pulsation effect. The micro-controller controls the motor revolutions by using the following algorithm:
a. the motor revolution is divided into a number of steps.
b. The micro-controller rotates the motor, sequentially from first step to the last step of each revolution, wherein each step or a group of steps are indicating the location of syringe actuator.
c. The pump output is controlled by three independent sensors which are comparing among them, results continuously.
d. The pump can detect the syringe size used using a linear potentiometer connected to the syringe holder 13. The syringe size can be sequentially detected during the pump work or can be used for calibration to obtain the syringe size and brand.

According to another aspect of the present invention, there is provided a dedicated flexible tube, with a pressure valve integrated, which will prevent free flow, of the syringe content if pressure is not generated by the syringe actuator.

### Detailed Description of Specific Embodiment

Reference is first being made to Fig 1 in which the mechanics of the syringe driver are shown. The motor 8 turns the screw axis 9 that initiate in both directions the movement of actuator 10.
The motor steps which initiate the actuator movement 10, are controlled by a magnet 1 and a hall effect sensor. Two independent sensors 6 are checking as well the motor movements.
The guiding bars 3 and 3A are stabilizing actuator 10, during movements, under pressure, but will as well conduct signals of sensor 2, indicating syringe location.

Figure 2 shows the pump 19, which can be connected to a docking station, not shown. The docking station having among other feature a LED display, is showing the most significant data on the LED display. Figure 2 shows three sensors that controls the syringe position number 15 controlling the syringe location in the syringe slot, number 13 senses the syringe diameter and number 2 the syringe plunger position . The operation led 18 will indicate the program status using a dual color led and number 12, a key board enable user to set/change the program.

## Claims

1. A syringe pump (19), for controllably administrating liquids to a patient from a syringe (16) wherein the driving mechanism of said pump (19) comprises:
[a] a motor (8);
[b] a screw axis (9), said screw axis (9) is rotationally coupled to said motor (8);
[c] a first guiding bar (3) and a second guiding bar (3A), wherein the former is disposed in parallel to the latter;
[d] an actuator (10), said actuator comprises:
[i] an actuator slot, said slot is adapted to accommodate the plunger (11) of said syringe (16);
[ii] a sensor (2), said sensor (2) is adapted to detect whether said plunger (11) of said syringe (16) is located within said slot as required;
[iii] wherein the screw axis initiates the movement of the actuator (10) in both directions;
said syringe pump (19) is **characterized by** that:
[e] said guiding bars (3, 3A) stabilizing said actuator (10) during movement along the guiding bars (3, 3A),and
[f] in parallel the signals of said sensor are transmitted via said guiding bars (3, 3A) to a controller of said motor (8).

2. The syringe pump according to any of the preceding claims, wherein said sensor (2) is a microswitch.

3. The syringe pump according to any of the preceding claims, **characterized in that** said sensor (2) is associated with said slot.

4. The syringe pump according to any of the preceding claims, wherein said sensor (2) is a microswitch forming an interruptible electrical circuit between said guiding bars (3, 3A) .

5. The syringe pump according to claim 4, wherein the interruptibility of said electrical circuit is indicative of whether said plunger (11) of said syringe (16) is located within said slot as required.

6. Syringe pump according to claim 1, **characterized in that** said guiding bars (3, 3A) prevent a rotational movement of said actuator (10) upon rotation of said motor (8) of said screw axis (9).

7. The syringe pump according to claim 6, **characterized in that** there is provided an encoder for controlling the location of said actuator (10).

## Patentansprüche

1. Spritzenpumpe (19) zum steuerbaren Verabreichen von Flüssigkeiten an einen Patienten aus einer Spritze (16), wobei der Antriebsmechanismus der Pumpe (19) Folgendes umfasst:
[a] einen Motor (8);
[b] eine Schraubenachse (9), wobei die Schraubenachse (9) an den Motor (8) drehgekoppelt ist;
[c] eine erste Führungsstange (3) und eine zweite Führungsstange (3A), wobei die Erstgenannte parallel zu der Letztgenannten angeordnet ist;
[d] ein Stellglied (10), wobei das Stellglied Folgendes umfasst:
[i] einen Stellgliedschlitz, wobei der Schlitz angepasst ist, um den Kolben (11) der Spritze (16) aufzunehmen;
[ii] einen Sensor (2), wobei der Sensor (2) angepasst ist, um zu erkennen, ob sich der Kolben (11) der Spritze (16) wie erforderlich in dem Schlitz befindet;
[iii] wobei die Schraubenachse die Bewegung des Stellglieds (10) in beide Richtungen einleitet;
wobei die Spritzpumpe (19) **dadurch gekennzeichnet ist, dass**:
[e] die Führungsstangen (3, 3A) das Stellglied (10) während der Bewegung entlang der Führungsstangen (3, 3A) stabilisieren, und
[f] parallel dazu die Signale des Sensors über die Führungsstangen (3, 3A) an ein Steuergerät des Motors (8) übertragen werden.

2. Spritzenpumpe nach einem der vorhergehenden Ansprüche, wobei der Sensor (2) ein Mikroschalter ist.

3. Spritzenpumpe nach einem der vorhergehenden Ansprüche, **dadurch gekennzeichnet, dass** der Sensor (2) dem Schlitz zugeordnet ist.

4. Spritzenpumpe nach einem der vorhergehenden Ansprüche, wobei der Sensor (2) ein Mikroschalter ist, der einen unterbrechbaren Stromkreis zwischen den Führungsstangen (3, 3A) bildet.

5. Spritzenpumpe nach Anspruch 4, wobei die Unterbrechbarkeit des Stromkreises ein Indikator dafür ist, ob sich der Kolben (11) der Spritze (16) wie erforderlich in dem Schlitz befindet.

6. Spritzenpumpe nach Anspruch 1, **dadurch gekennzeichnet, dass** die Führungsstangen (3, 3A) eine Drehbewegung des Stellglieds (10) bei Drehung des Motors (8) der Schraubenachse (9) verhindern.

7. Spritzenpumpe nach Anspruch 6, **dadurch gekennzeichnet, dass** ein Geber zum Steuern der Position des Stellglieds (10) vorgesehen ist.

## Revendications

1. Pompe à seringue (19) pour l'administration contrôlée de liquides à un patient par une seringue (16), le mécanisme d'entraînement de ladite pompe (19) comprenant :
[a] un moteur (8) ;
[b] un axe fileté (9), ledit axe fileté (9) étant couplé par rotation audit moteur (8) ;
[c] une première barre de guidage (3) et une deuxième barre de guidage (3A), la première étant disposée parallèlement à la dernière ;
[d] un actionneur (10), ledit actionneur comprenant :
[i] une fente d'actionneur, ladite fente étant adaptée pour recevoir le piston (11) de ladite seringue (16) ;
[ii] un capteur (2), ledit capteur (2) étant adapté pour détecter si ledit piston (11) de ladite seringue (16) est situé au sein de ladite fente conformément à la spécification ;
[iii] l'axe fileté amorçant le déplacement de l'actionneur (10) dans les deux sens ;
ladite pompe de seringue (19) étant **caractérisée en ce que** :
[e] lesdites barres de guidage (3, 3A) stabilisent ledit actionneur (10) au cours du déplacement des barres de guidage (3, 3A), et
[f] parallèlement les signaux dudit capteur sont transmis par le biais desdites barres de guidage (3, 3A) à une commande dudit moteur (8).

2. Pompe à seringue selon une quelconque des revendications précédentes, ledit capteur (2) étant un microcontact.

3. Pompe à seringue selon une quelconque des revendications précédentes, **caractérisée en ce que** ledit capteur (2) est associé avec ladite fente.

4. Pompe à seringue selon une quelconque des revendications précédentes, ledit capteur (2) étant un microcontact formant un circuit électrique interruptible entre lesdites barres de guidage (3, 3A).

5. Pompe à seringue selon la revendication 4, l'interruptibilité dudit circuit électrique indiquant si ledit piston (11) de ladite seringue (16) est situé au sein de ladite fente, conformément aux spécifications.

6. Pompe à seringue selon la revendication 1, **caractérisée en ce que** lesdites barres de guidage (3, 3A) empêchent un mouvement rotatoire dudit actionneur (10) lors de la rotation dudit moteur (8) dudit axe fileté (9).

7. Pompe à seringue selon la revendication 6, **caractérisée en ce qu'**est incorporé un codeur pour le contrôle de la localisation dudit actionneur (10).
